# EUROPEAN PATENT SPECIFICATION

(11) **EP 1 782 746 B1**
(45) Date of publication and mention of the grant of the patent: **19.12.2012**
(21) Application number: 06022484.7
(22) Date of filing: 27.10.2006
(51) Int. Cl.: A61B 19/08

(54) **A disposable surgical drape**
Wegwerfbares Chirurgisches Abdecktuch
Drap chirurgical jetable

(30) Priority: 03.11.2005 IT MI20050385 U
(43) Date of publication of application: 09.05.2007
(73) Proprietor: N.G.C. Medical S.p.A., 22060 Novedrate CO (IT)
(72) Inventor: Cremascoli, Eugenio, 22060 Novedrate (COMO) (IT); Pini, Patrizia, 22060 Novedrate (COMO) (IT)
(74) Representative: Cicogna, Franco

(56) References cited:
- WO-A-01/30258
- WO-A2-02/41800
- US-A- 2 593 121
- US-A- 3 856 005
- US-A- 4 873 997
- US-A- 5 222 507
- US-A- 5 383 476
- US-A1- 2003 188 753

## Description

### BACKGROUND OF THE INVENTION

The present invention relates to a patient covering disposable surgical drape for angiographic or electrophysiologic procedures.

As is known, disposable patient covering surgical drapes, to be used in angiographic or cardiac catheterization procedures, with a percutaneous access, or in electrophysiologic procedures, are designed to perform several functions.

The main function of the surgical drape, which is used in a sterile condition, is that of holding a sterile field of operation during the procedure being performed at the access region.

In other words, the surgical drape must substantially cover the patient, to provide a barrier between a sterile environment and a non sterile environment.

Said surgical drape, in particular, is adapted to perform its desired functions since it is provided with antibacteric, fluid and blood resistant, tears resistant properties, and since, moreover, it does not include free fibers and provides a high comfort for the patient.

Prior surgical drapes, as commercially available, have a plurality of regions which are impermeable with respect to the patient and adapted to provide absorption properties near the opening and surgical processing zones; moreover, said prior drapes are also provided with antibacteric characteristics.

The access regions providing access to the patient body are at first disinfected and then covered by a plastic adhesive film thereon and therethrough the surgical cut is performed.

The film contacts the patient skin to insulate the access region from possible contaminations.

Available disposable surgical drapes are conventionally made of a non woven fabric material, plastic or paper material and have provided generally satisfactory results; however, it has been found that said prior drapes would be susceptible to improvements, in particular with respect to their patient insulating properties, their outside environment insulating properties, and their liquid confining properties.

The document US 2003/188753 A discloses a patient covering disposable surgical drape for angiographic procedures, comprising: a central region made of a liquid impermeable material; wherein said central region includes surgical access sites and additional (stiffening) portions having high absorption properties; and adjacent side regions made of optically transparent material.

### SUMMARY OF THE INVENTION

Accordingly, the aim of the present invention is to overcome the above mentioned problems by providing a surgical or patient covering drape, of a single-use or disposable type, for angiographic or electrophysiologic procedures, allowing to obtain a full covering of the patient, while holding in a sterile condition the patient neck region, which is accordingly used for engaging therein a central venous catheter.

Within the scope of the above mentioned aim, a main object of the present invention is to provide such a surgical drape, designed for preventing the surgeon from accidentally contacting the patient blood.

A further object of the present invention is to provide such a disposable patient covering surgical drape which, owing to its specifically designed constructional features, is very reliable and safe in operation.

Yet another object of the present invention is to provide such a surgical drape which can be easily made and which, moreover, is very competitive from a mere economic standpoint.

According to one aspect of the present invention, the above mentioned aim and objects, as well as yet other objects, which will become more apparent hereinafter, are achieved by a patient covering disposable surgical drape for angiographic or electrophysiologic procedures, comprising a central impermeable and breathable region, defining access regions adjoining with optically transparent side regions, wherein said surgical towel comprises moreover, at said central portion, at least further surgical drape portions having high absorption properties, and corresponding wing portions for arrangement at the sides of the patient neck, according to the appended claim 1.

### BRIEF DESCRIPTION OF THE DRAWINGS

Further characteristics and advantages of the present invention will become more apparent hereinafter from the following detailed disclosure of a preferred, though not exclusive, embodiment of a patient covering disposable surgical drape for angiographic or electrophysiologic procedures, which is illustrated, by way of an indicative, but not limitative, example in the accompanying drawings, where:

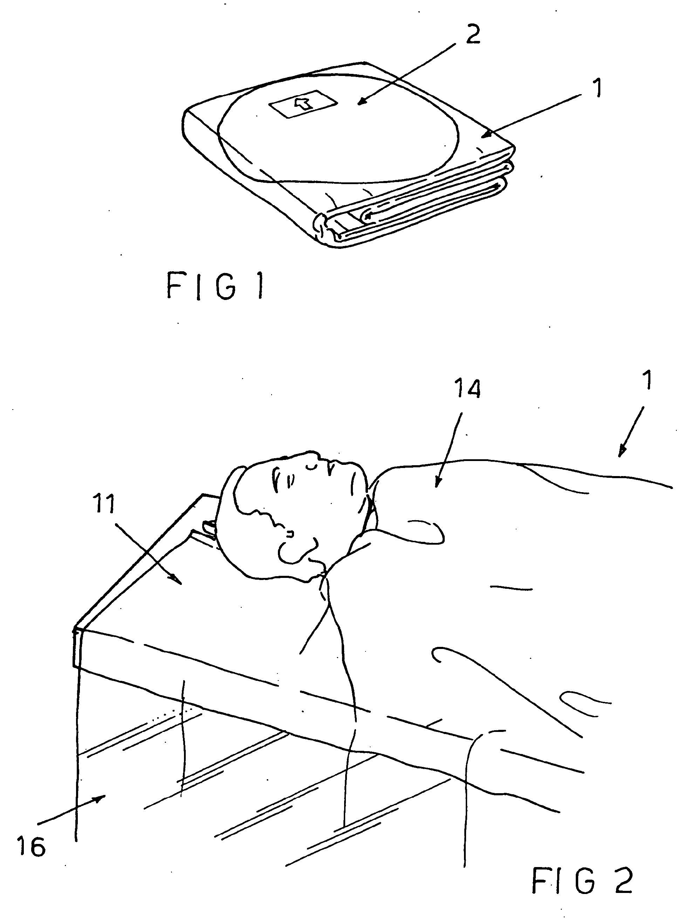

Figure 1 shows a surgical drape according to the present invention in a folded condition thereof;
Figure 2 shows the surgical drape of the present invention applied to a patient and clearly shows the drape wings arranged at the sides of the patient neck;

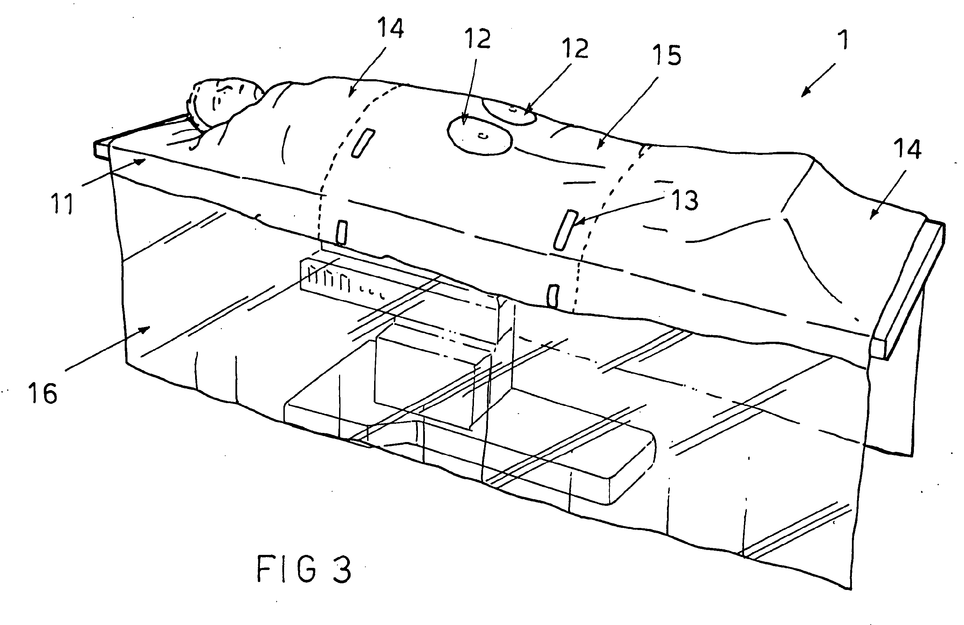

Figure 3 is a schematic view showing the surgical drape applied to the patient;

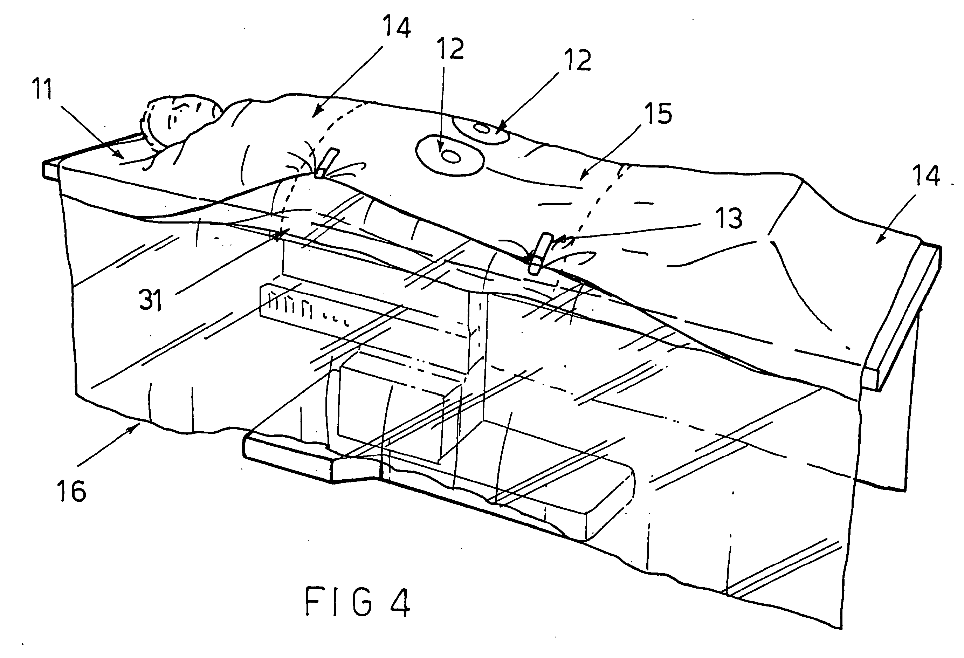

Figure 4 shows a pocket arrangement formed on the outer surface of the surgical drape;

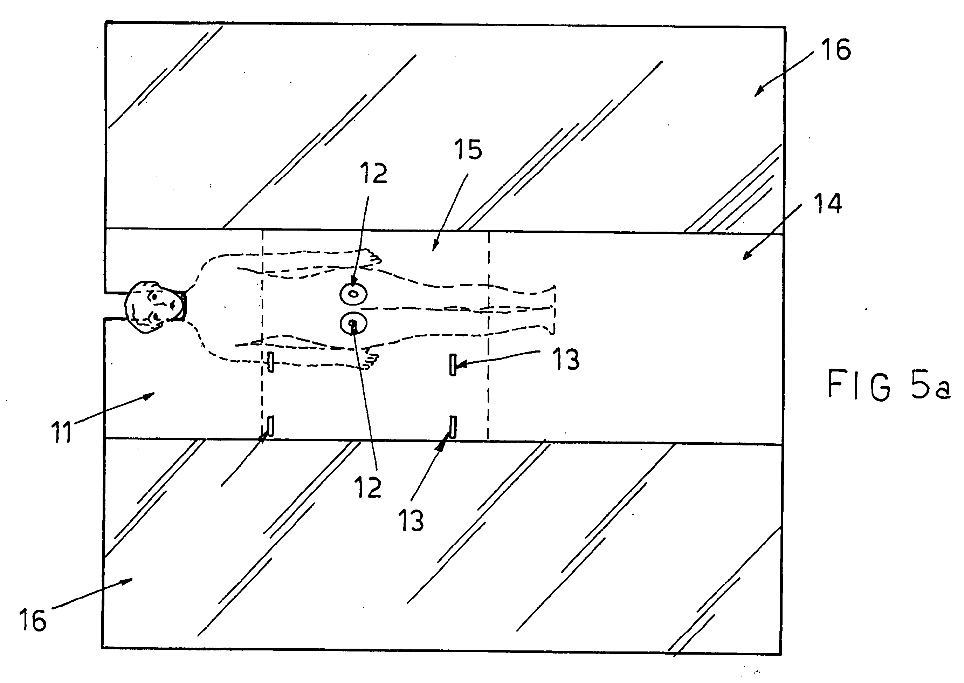

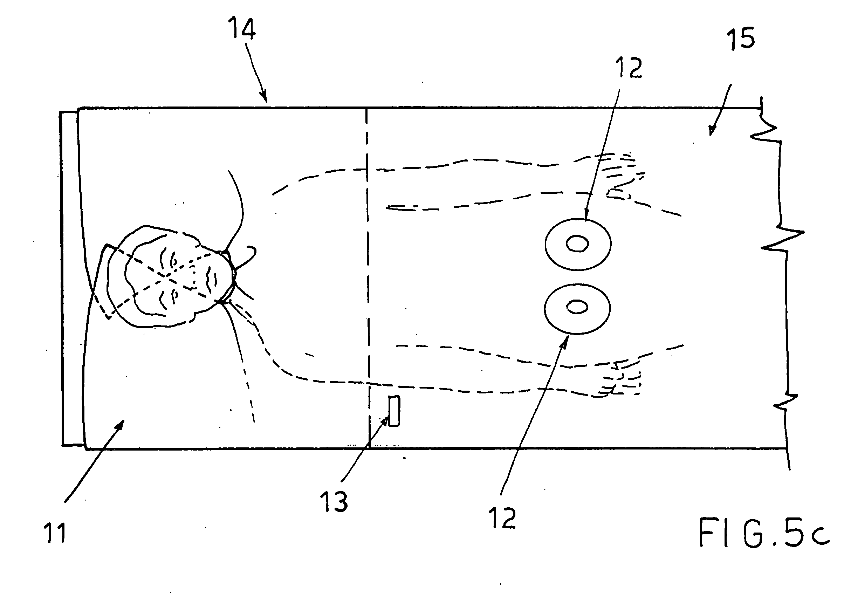

Figures 5a, 5b and 5c schematically show the surgical drape, as seen from the top and in a distended or extended position thereof;
Figure 6 shows a detail of window arrangements formed through the subject surgical drape; and
Figure 7 shows an exemplary surgical drape designed for performing electrophysiologic operations

### DESCRIPTION OF THE PREFERRED EMBODIMENT

With reference to the number references of the above mentioned figures, the patient covering disposable surgical drape for angiographic or electrophysiologic procedures according to the present invention, which has been generally indicated by the reference number 1, comprises window arrangements, indicated by the reference number 2, which are provided to be located at the cardiac catheter insertion region.

More specifically, said window arrangements may have an oval configuration or any other desired and suitable shape, and are characterized by the provision of a cut-through plastic film.

Said cut-through plastic film has adhesive properties and is applied to the skin of the patient.

Said film is protected by a removable paper material, which is detached from the film to cause the film to adhere to the patient skin, as a sticking plaster.

As shown, the window arrangements are located at the patient groin region, to allow the femoral artery to be easily accessed.

In particular, said window arrangements are arranged at the right and left leg of the patient, to allow the two types of accesses to be easily performed.

The right or left accesses may be programmed, but they must be both present to allow a possibility of performing an alternating introduction of the catheter, if required.

Other access types comprise a radial access or brachial access.

In this case, the window arrangements will be located near the patient wrists or elbows.

The window arrangement 12 can be made of a low density polyethylene material, provided with an adhesive or sticking side.

The adhesive or sticking side will be pressure applied, and it must be biologically acceptable.

The adhesive part or side is protected by a suitable protective material, for example silicone processed paper, which will be removed in operation.

The surgical drape comprises a non adhesive central region 14, which is of an impermeable and breathable type.

The surgical drape has the function of protecting the patient from liquids, such as blood, ejected during the surgical procedure or operation and, in addition, to properly insulate the patient body unsterile regions from the operating field.

A satisfactory surgical drape must also have breathing and draping properties, to provide the patient with a good comfort.

A main feature of the invention is that at the central region 14, i.e. at the head end portions thereof, a longitudinal opening is provided defining two wings 11 to be arranged at the side of the patients to fully cover the patient, while improving the sterile properties of the operating field.

The surgical drape, at the patient foot portion, projects for a comparatively high extension from the patient feet.

This portion is usually used for covering the part of the radiographic table which can be used as a preparing surface for preparing the operational devices or tools used during the surgical procedure.

Near or joining the central region, are provided further side regions 16, which are advantageously optically transparent, to allow the driving console to be properly operated for driving the radiographic table while preserving the desired operating field sterile properties, by properly insulating the operating field from the control console.

The above mentioned side regions will be generally made of a polyethylene material.

Above the mentioned central region, at least a high absorbance portion 15 is provided.

Said high absorbance regions are located near the mentioned window arrangements, i.e. near the surgical cut-through regions and along the patient legs, to properly absorb the patient blood which is ejected during the operation procedures performed on the catheter, such as the removal of the catheter guide or the insertion of catheters of a comparatively long length.

In particular, a high absorbance region of the surgical drape is arranged near the area where the surgeon will be operating.

This region comprises tearable strip portion 13, of a male and female type, to provide a pocket arrangement by folding the surgical drape.

In this region, the surgical drape is advantageously stiffened or reinforced and made impermeable, to provide a blood holding pocket or bag, for preventing blood from being ejected toward the patient.

This modular pocket, having two operating positions, will operate to properly collect the patient ejected blood, to absorb the latter and prevent it from contacting the surgeon or operator, in addition to providing a sterile pocket for arranging therein surgical tools to properly free the operating field.

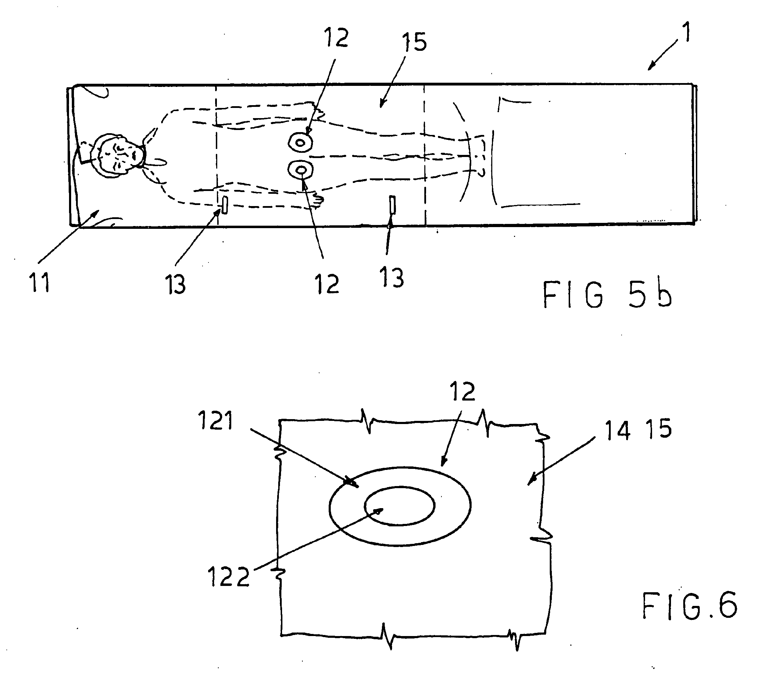

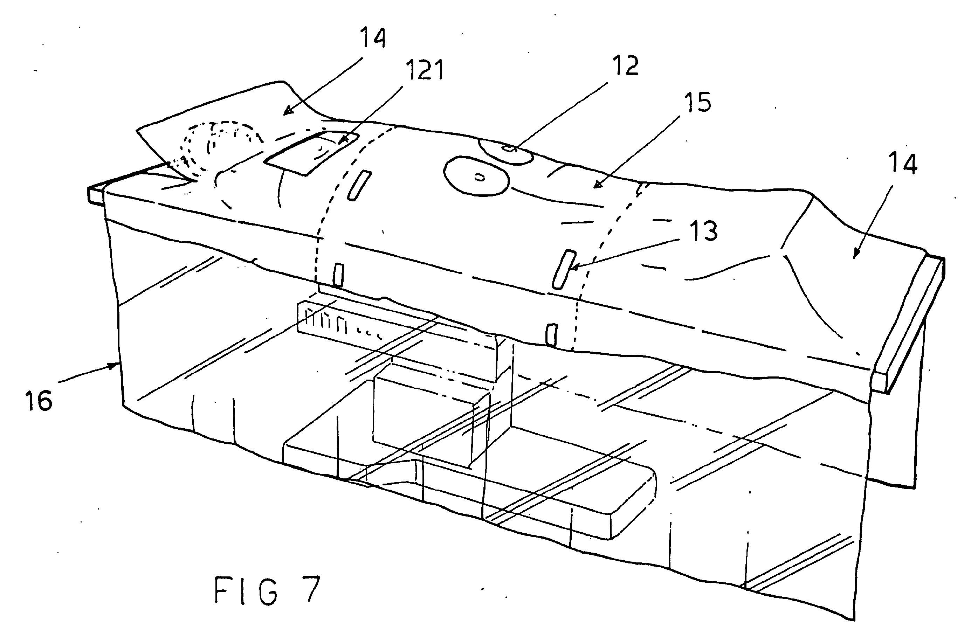

As shown in figures 6 and 7, an adhesive cloth piece, indicated by the reference number 121, is moreover arranged in said window arrangements, to practically encompass a cut-through hole 122.

Figure 7 shows a surgical drape for performing an electrophysiologic operation, with a window for an implantation of a pace-maker.

In such drape, a window is moreover provided to allow access to the subclavian vein, preferably the left one, and for properly locating the electro-stimulating device.

In such a procedure it is possible to simultaneously use a femoral window and a thorax window, for example for locating a temporarily pacemaker, during the replacement of the implantable pacemaker, and during the location of the implantable defibrilators.

For such exemplary surgical drape, the drape wings for covering the patient shoulders are omitted, since a separating element is frequently used for allowing the surgical drape to pass therethrough, thereby preventing the patient from seeing his/her thorax.

The absorbing pocket, on the other hand, can be also herein used and it is preferably arranged at the left.

From the above disclosure it should be apparent that the invention fully achieves the intended aim and objects.

In fact, the invention provides a surgical drape having very satisfactory operation and properties, which allows to optimize all the operation procedures or steps.

The invention, as disclosed, is susceptible to several modifications and variations, all of which will come within the scope of the appended claims.

In practicing the invention, the used materials, provided that they are compatible to the intended application, and the contingent size and shapes, can be any, depending on requirements.

## Claims

1. A patient covering disposable surgical drape (1) for angiographic or electrophysiologic procedures, allowing to obtain a full covering of the patient while holding in a sterile condition the patient's neck region for allowing a central venous catheter to be engaged in said neck region, and for preventing a surgeon from accidentally contacting a patient blood, comprising a central region (14) made of a liquid impermeable and breathable material, wherein said central region (14) includes surgical access sites (12) and an adjacent region (16) made of an optically transparent material, said surgical drape further comprising over said central region (14) additional portions (15) having high absorption properties and at the head end portion of said central region a longitudinal opening defining two wing portions (11) for arrangement at the sides of the patient's neck and for holding in a sterile condition the neck region, and wherein at one of said highly absorbing portions said surgical drape (1) is stiffened and made impermeable to liquids, and wherein one of said highly absorbing portions further comprises a tearable strip portion (13) of a male and female type adapted to provide a modular bifunctional pocket for collecting blood for preventing blood from contacting the surgeon or for housing surgical tools therein, upon folding said surgical drape.

2. A surgical drape (10), according to claim 1, **characterized in that** said surgical access sites (12) comprise an adhesive plastic film (121) protected by a removable paper material.

3. A surgical drape (10), according to claim 2, **characterized in that** said plastic film (121) has a central opening (122) for facilitating a surgical cutting operation.

## Patentansprüche

1. Patienten-bedeckendes, wegwerfbares chirurgisches Abdecktuch (1) für angiographische oder elektrophysiologische Verfahren, welches es ermöglicht, eine vollständige Abdeckung des Patienten zu erhalten, während der Halsbereich des Patienten in einem sterilen Zustand gehalten wird, um zu ermöglichen, dass ein zentraler Venenkatheter in den Halsbereich eingesetzt werden kann, und um zu verhindern, dass ein Chirurg versehentlich mit dem Patientenblut in Kontakt kommt, welches einen Mittelbereich (14), der aus einem flüssigkeitsundurchlässigen und atemfähigen Material hergestellt ist, wobei der Mittelbereich (14) chirurgische Zugangsöffnungen (12) enthält, und einen angrenzenden Bereich (16) aufweist, der aus einem optisch transparenten Material hergestellt ist, wobei das chirurgische Abdecktuch ferner über dem Mittelbereich (14) zusätzliche Abschnitte (15) mit hohen Absorptionseigenschaften und am kopfseitigen Endabschnitt des Mittelbereichs eine längliche Öffnung aufweist, die zwei Flügelabschnitte (11) zur Anordnung an den Seiten des Patientenhalses und zum Halten des Halsbereichs in einem sterilen Zustand definieren und wobei an einem der äußerst absorptionsfähigen Abschnitte das chirurgische Abdecktuch (1) versteift und undurchlässig für Flüssigkeiten gemacht ist und wobei einer der äußerst absorptionsfähigen Abschnitte ferner einen Abschnitt mit Aufreißstreifen (13) eines Innenteil- und Außenteil-Typs aufweist, der angepasst ist, beim Falten des chirurgischen Abdecktuchs eine modulare, bifunktionelle Tasche für das Sammeln von Blut zum Verhindern, dass der Chirurg mit Blut in Kontakt kommt, oder zum Unterbringen von chirurgischen Instrumenten darin bereitzustellen.

2. Chirurgisches Abdecktuch (10) gemäß Anspruch 1, **dadurch gekennzeichnet, dass** die chirurgischen Zugangsöffnungen (12) eine klebende Kunststofffolie (121) aufweisen, die durch ein entfernbares Papiermaterial geschützt ist.

3. Chirurgisches Abdecktuch (10) gemäß Anspruch 2, **dadurch gekennzeichnet, dass** die Kunststofffolie (121) eine zentrale Öffnung (122) zum Ermöglichen eines chirurgischen Schneidvorgangs aufweist.

## Revendications

1. Drap chirurgical jetable (1) destiné à recouvrir un patient dans les cas de procédures angiographiques ou électrophysiologiques, permettant d'obtenir le recouvrement complet du patient tout en maintenant à l'état stérile la région du cou du patient afin de permettre l'introduction d'un cathéter veineux central dans ladite région du cou et d'empêcher un chirurgien d'entrer en contact, accidentellement, avec le sang du patient, comprenant une région centrale (14) composée d'un matériau imperméable aux liquides et perméable à l'air, dans lequel ladite région centrale (14) inclut des sites d'accès chirurgical (12) et une région adjacente (16) composée d'un matériau optiquement transparent, ledit drap chirurgical comprenant en outre, par-dessus ladite région centrale (14) des parties supplémentaires (15) présentant des propriétés d'absorption élevée et, au niveau de la partie d'extrémité de tête de ladite région centrale, une ouverture longitudinale définissant deux parties d'aile (11) destinées à être agencées au niveau des côtés du cou du patient et à maintenir à l'état stérile la région du cou, et dans lequel, au niveau de l'une desdites parties présentant des propriétés d'absorption élevée, ledit drap chirurgical (1) est renforcé et rendu imperméable aux liquides, et dans lequel l'une desdites parties présentant des propriétés d'absorption élevée comprend en outre une partie de bande pouvant être déchirée (13) d'un type male et femelle conçue pour fournir une poche modulaire bi-fonctionnelle destinée à recueillir le sang, afin d'empêcher le sang d'entrer en contact avec le chirurgien, ou à abriter, dans celles-ci, des outils chirurgicaux, lors du pliage dudit drap chirurgical.

2. Drap chirurgical (10) selon la revendication 1, **caractérisé en ce que** lesdits sites d'accès chirurgical (12) comprennent un film en plastique adhésif (121) protégé par un papier amovible.

3. Drap chirurgical (10) selon la revendication 2, **caractérisé en ce que** ledit film de plastique (121) possède une ouverture centrale (122) destinée à faciliter une opération chirurgicale invasive.
